# EUROPEAN PATENT APPLICATION

(11) **EP 3 235 416 A1**
(43) Date of publication of application: **25.10.2017**
(21) Application number: 15869948.8
(22) Date of filing: 14.12.2015
(51) Int. Cl.: A61B 1/00, A61B 1/04, G02B 23/24

(54) **ENDOSCOPIC APPARATUS**

(30) Priority: 15.12.2014 JP 2014253507
(71) Applicant: Olympus Corporation, Hachioji-shi Tokyo 192-8507 (JP)
(72) Inventor: UCHIDA, Taiji, Hachioji-shi Tokyo 192-8507 (JP); USHIFUSA, Hiroyuki, Hachioji-shi Tokyo 192-8507 (JP)
(74) Representative: Gunzelmann, Rainer
(86) International application number: PCT/JP2015/084979
(87) International publication number: WO 2016/098743

(57) **Abstract**

In order to provide a technology that makes it possible to set the detection sensitivity of a touch panel of an endoscopic device appropriately according to the usage environment of an endoscopic system, a touch panel 81 is provided in an endoscopic video processor 2. A touch-panel-sensitivity setting device 25 sets the detection sensitivity of a touch manipulation performed on a touch panel 85 on the basis of input user-setting information.

## Description

### Technical Field

The present invention relates to an endoscopic device that includes a capacitive touch panel.

### Related Art

In an endoscopic system, touch panels are used in various devices that constitute the system in order for a user to perform a manipulation more easily (see, for example, Patent Documents 1 and 2). For example, when a capacitive touch panel is used, a change in capacitance (charge) due to the surface of the touch panel being touched with a finger is detected so as to detect a touched position.

A user of an endoscopic system may have to manipulate a touch panel when he/she is wearing gloves, and when a capacitive touch panel is used, its reaction is different depending on whether the user touches the touch panel with bare hands or when he/she is wearing gloves. For example, a technology has been disclosed that sets a manipulation input device on the basis of a sensitivity setting parameter of the touch panel for each type of contact with the capacitive touch panel, such as according to whether the contact has been made with bare hands or in a state in which gloves are worn (see, for example, Patent Document 3) . According to such a technology, the sensitivity of a touch panel is set appropriately by a user performing a manipulation to select one of the types of contacts.

### Citation List

### Patent Document

Patent Document 1: Japanese Laid-open Patent Publication No. 2004-165728
Patent Document 2: Japanese Laid-open Patent Publication No. 2005-329130
Patent Document 3: Japanese Laid-open Patent Publication No. 2012-173749

### Summary of Invention

### Technical Problem

When a touch panel is provided in a housing that constitutes an endoscopic system such as an endoscopic video processor or a light source device, an optimal sensitivity setting has to be performed according to the individual differences in the touch panel sensitivity of a user or according to whether a user is wearing gloves (that is, whether the user is a doctor specializing in internal medicine or in surgery). However, this is not considered in the technologies disclosed in the patent documents described above. Thus, a user that uses an endoscopic system has had to perform a sensitivity setting of a touch panel before he/she uses the endoscopic system in each case. For a user, it is preferable that the detection sensitivity of a touch panel be more easily set appropriately according to, for example, the usage environment of a system.

An object of the present invention is to provide a technology that makes it possible to set the detection sensitivity of a touch panel of an endoscopic device appropriately according to, for example, the usage environment of an endoscopic system.

### Solution to Problem

An endoscopic device according to an aspect of the present invention includes an endoscopic video processor, a touch panel provided in the endoscopic video processor, and a touch-panel-sensitivity setting device that sets, on the basis of input identification information, a detection sensitivity of a touch manipulation performed on the touch panel.

### Advantageous Effects of Invention

According to embodiments of the present invention, the sensitivity of a touch panel of an endoscopic device is set appropriately according to, for example, the usage environment of an endoscopic system.

### Brief Description of Drawings

FIG. 1 illustrates a configuration of an endoscopic system that includes an endoscopic video processor according to a first embodiment;
FIG. 2 is a diagram that explains a correspondence relationship between the field of application of an endoscope according to a first embodiment and the detection sensitivity of a touch panel;
FIG. 3 illustrates a configuration of the endoscopic system that includes the endoscopic video processor according to a second embodiment; and
FIG. 4 is a diagram that explains a method for determining a false detection that is executed by the processor.

### Description of Embodiments

Embodiments will now be described with reference to the drawings.

### <First Embodiment>

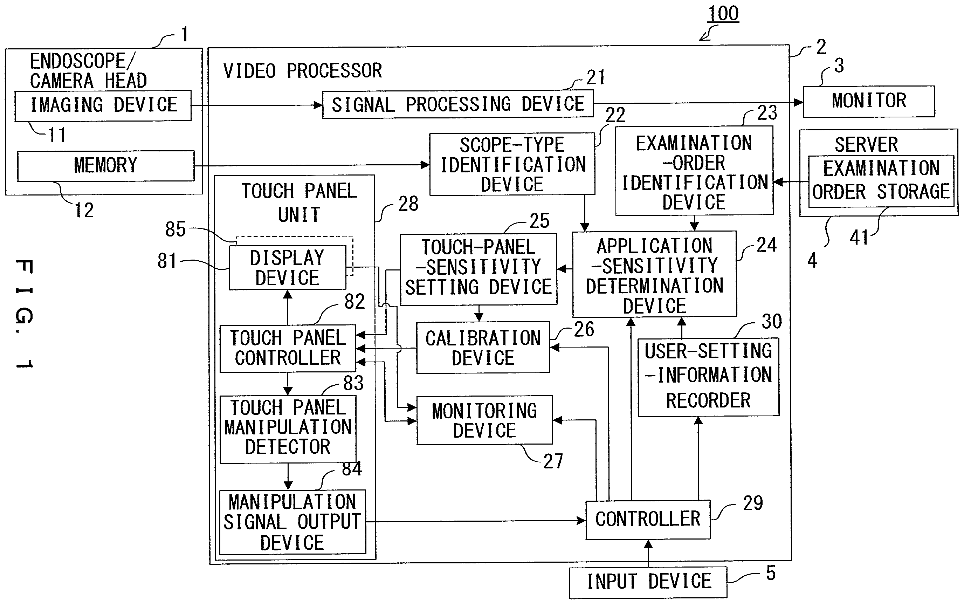

FIG. 1 illustrates a configuration of an endoscopic system that includes an endoscopic video processor according to the present embodiment. An endoscopic system 100 illustrated in FIG. 1 includes an endoscope 1, an endoscopic video processor (hereinafter referred to as a processor) 2, a monitor 3, and a server 4.

The endoscope 1 captures, using an imaging device 11, an image of the inside of the body cavity of a patient, that is, an image of a subject, and transmits a video signal obtained by the image capturing to the processor 2. The endoscope 1 may be provided with a camera head when it is used during a surgery. A memory 12 of the endoscope 1 holds a variety of information such as a scope ID (identification) that identifies a type of scope.

The processor 2 includes a signal processing device 21, a touch panel unit 28, a controller 29, a user-setting-information recorder 30, an application-sensitivity determination device 24, a touch-panel-sensitivity setting device 25, a scope-type identification device 22, an examination-order identification device 23, a calibration device 26, and a monitoring device 27. The processor 2 performs necessary processing on a video signal input from the endoscope 1, and outputs the processed video signal to an external device. FIG. 1 illustrates the monitor 3 as an example of an output destination.

In the processor 2, the signal processing device 21 performs necessary processing, such as image processing, on a video signal input from the endoscope 1. The endoscopic system 100 of FIG. 1 outputs, to the monitor 3, the video signal output from the signal processing device 21. The monitor 3 displays an endoscopic video on the basis of the video signal received from the processor 2.

The processor 2 detects a position pressed by a user on a touch panel 85 using the touch panel unit 28, and receives an input of a manipulation instruction corresponding to the detected position from among various manipulation instructions. The touch panel unit 28 includes a display device 81, the touch panel 85, a touch panel manipulation detector 83, a manipulation signal output device 84, and a touch panel controller 82.

The display device 81 displays various screens such as a menu screen and a parameter setting screen. The touch panel controller 82 controls an operation of each component in the touch panel unit 28, and detects a coordinate of a position pressed by the user on the touch panel 85 provided in the display device 81. On the basis of the coordinate of a position pressed on the touch panel 85, the touch panel manipulation detector 83 detects a manipulation instructed by the user, the coordinate of a position being detected by the touch panel controller 82. The touch panel manipulation detector 83 reports the content of the detected manipulation instructed by the user to the manipulation signal output device 84. The manipulation signal output device 84 outputs, to the controller 29, a manipulation signal according to the report from the touch panel manipulation detector 83.

The controller 29 controls each component that constitutes the processor 2 and communicates with each device that constitutes the endoscopic system 100, so as to transmit and receive a video signal and other various signals. When it receives a manipulation signal from the manipulation signal output device 84 of the touch panel unit 28, the controller 29 transmits, to each of the components, an instruction signal that instructs them to perform processing corresponding to the manipulation signal.

### (Setting of Sensitivity of Touch Panel)

In the processor 2 according to the present embodiment, the sensitivity upon detecting a touch manipulation of a user in the touch panel unit 28 is set according to whether the endoscope 1 is used in internal medicine or in surgery. In regard to this matter, the controller 29 controls each component involved in processing of setting the sensitivity of the touch panel 85 according to the field of application of the endoscope 1.

Specifically, the controller 29 causes the application-sensitivity determination device 24 to perform processing of determining the detection sensitivity of the touch panel 85 at a predetermined timing such as at startup or upon starting an examination. At this point, the controller 29 reads user-setting information associated with a user from the user-setting-information recorder 30, and reports the read user-setting information to the application-sensitivity determination device 24.

The application-sensitivity determination device 24 determines, according to an instruction from the controller 29, whether "internal medicine" or "surgery" is set to be a field of application in the user-setting information read from the user-setting-information recorder 30. Then, the application-sensitivity determination device 24 reports, to the touch-panel-sensitivity setting device 25, the field of application that is set in the user-setting information.

The touch-panel-sensitivity setting device 25 sets the detection sensitivity in the touch panel manipulation detector 83 of the touch panel unit 28 according to the field of application reported from the application-sensitivity determination device 24. For example, the detection sensitivity is set to "high" when "surgery" is set to be a field of application, and the detection sensitivity is set to "low" when "internal medicine" is set to be a field of application. In the example, for example, the detection sensitivity is set to "medium" when there is no report on the field of application from the application-sensitivity determination device 24.

The detection sensitivity on the touch panel 85 depends on which value is set as a reference value of capacitance. The detection sensitivity is low if a relatively high value is set as a reference value of capacitance, and the detection sensitivity is high if a relatively low value is set as the reference value. Thus, the touch-panel-sensitivity setting device 25 of the processor 2 sets the reference value of capacitance as appropriate according to the field of application reported from the application-sensitivity determination device 24, the reference value of capacitance being within a predetermined range determined by, for example, a specification of the touch panel unit 28. Then, the touch-panel-sensitivity setting device 25 reports the set reference value to the touch panel unit 28. According to the report received from the touch-panel-sensitivity setting device 25, the touch panel controller 82 sets the reference value of capacitance upon detecting a touch on the touch panel 85. When the capacitance of the touch panel 85 is greater than or equal to a threshold, the touch-panel-sensitivity setting device 25 determines that the position has been touch-manipulated, wherein the set reference value is the threshold.

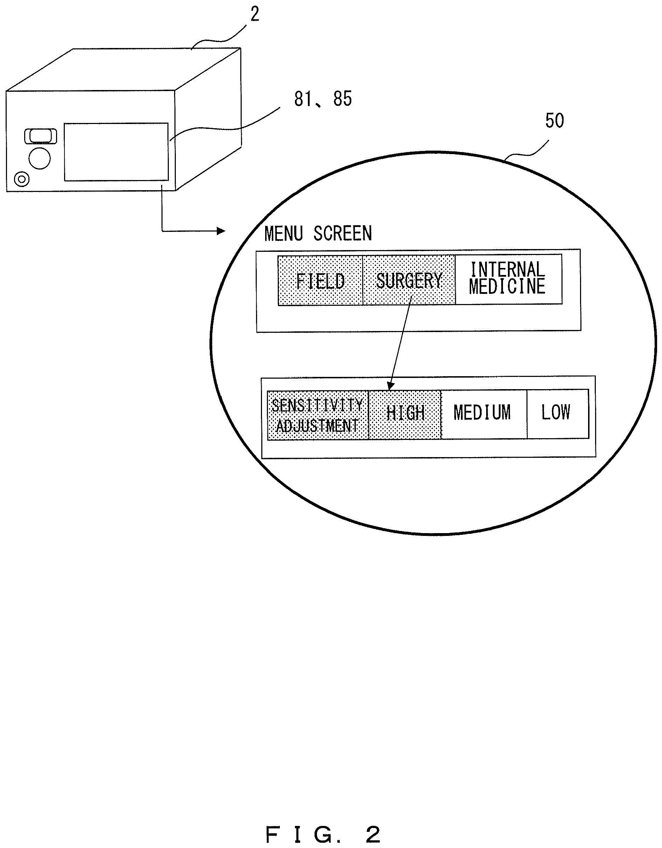

The processor 2 according to the present embodiment also makes it possible to change the detection sensitivity of the touch panel 85 as appropriate according to, for example, an instruction from a user after the detection sensitivity of the touch panel 85 is set at a timing such as at startup or upon starting an examination. This is described with reference to FIG. 2.

FIG. 2 is a diagram that explains a correspondence relationship between the field of application of the endoscope 1 according to the present embodiment and the detection sensitivity upon detecting a touch using the touch panel controller 82. FIG. 2 illustrates a perspective view of the processor 2, and a screen 50 displayed on a display part 81 provided on the front surface of a body of the processor 2.

As described above, at startup or upon starting an examination, the processor 2 sets the detection sensitivity of the touch panel 85 on the basis of the user-setting information recorded in the user-setting-information recorder 30. On the other hand, for example, there is a possibility that a user will want to have the detection sensitivity set in the processor 2 made higher or lower when the touch panel 85 is used after the detection sensitivity is set. Thus, according to the present embodiment, even when a change in the detection sensitivity of the touch panel 85 is instructed by the user through, for example, the screen 50 displayed on the display part 81 or an input device 5 of FIG. 1, the controller 29 also causes the application-sensitivity determination device 24 to perform processing of determining the detection sensitivity of the touch panel 85.

A keyboard, a pointing device, and a button provided on the body of the processor 2 are examples of the input device 5 of FIG. 1.

When it confirms, through, for example, the screen 50 displayed on the display part 81 or a manipulation performed by the input device 5, an instruction to change the detection sensitivity of the touch panel 85, the controller 29 reports to the application-sensitivity determination device 24 on the field of application input through, for example, the input device 5. The application-sensitivity determination device 24 reports, to the touch-panel-sensitivity setting device 25, the reported field of application according to the report from the controller 29. The subsequent operation is as described above.

Accordingly, a configuration in which the detection sensitivity of the touch panel 85 can be changed according to an instruction from a user makes it possible to set the detection sensitivity of the touch panel 85 for each user more appropriately.

As described above, in the example, a relatively higher detection sensitivity is set when the field of application is surgery, compared to when the field of application is internal medicine. The reason is that, usually, when the field of application is surgery, a user is wearing rubber gloves when touch-manipulating the touch panel 85. It is difficult to detect a touch manipulation on the capacitive touch panel 85 if the user is wearing an insulator such as a rubber glove. However, when the field of application is surgery, if the detection sensitivity is set to be relatively high, it is possible for the user to manipulate the touch panel 85 speedily and easily without the maneuverability being affected due to it being possible to use in a state in which rubber gloves are worn, that is, due to the field of application of the endoscope 1 or the facilities in which the endoscope 1 is used.

Alternatively, the detection sensitivity may be set to a maximum value that can be set when surgery has been set to be a field of application. Likewise, such a configuration also permits a user to manipulate the touch panel 85 speedily and easily without the maneuverability being affected due to the field of application of the endoscope 1 or the facilities in which the endoscope 1 is used.

When the field of application is internal medicine, it is preferable that the detection sensitivity be set to be relatively low, as in the example. The reason is that it is often the case that a medicine and the like is used in an examination or a medicine is placed on, for example, a trolley provided with the processor 2 when it is used in internal medicine. In other words, a medicine placed on a trolley may spill out so that the medicine that has spilt out adheres to the display part 81 of the processor 2. If the detection sensitivity is set to be high for use in internal medicine, there is a good possibility that an adhered medicine will also be detected as a touch manipulation. Thus, if the detection sensitivity is set to be respectively low, it is possible to effectively prevent malfunction caused by recognizing, as a touch manipulation, a medicine or the like that has become adhered.

As described above, in the processor 2 according to the present embodiment, the detection sensitivity of the touch panel 85 is set appropriately for each user. For example, when the field of application is surgery, the detection sensitivity of the touch panel 85 is set to be relatively higher, compared to when the field of application is internal medicine. A user may manipulate the touch panel 85 wearing rubber gloves for a surgery or the like when the field of application is surgery, and the detection sensitivity of the touch panel 85 is set appropriately according to the usage, which permits the user to manipulate the touch panel 85 speedily and easily. Accordingly, the processor 2 according to the present embodiment permits a user to simply set an appropriate detection sensitivity of the touch panel 85 according to, for example, the usage environment of the endoscopic system 100.

### (Modification 1)

A method other than the method for performing a setting on the basis of user-setting information stored in the user-setting-information recorder 30 and the method for performing a setting on the basis of information input by a user through the touch panel 85 or the input device 5 may be used as a method for setting the field of application of the endoscope 1. For example, when it is possible to confirm whether the endoscope 1 is going to be used in internal medicine or in surgery, the field of application may be set on the basis of the recognition.

Specifically, it may be determined whether the endoscope 1 is going to be used in internal medicine or in surgery according to the type of scope of the endoscope 1 or the content of an examination order.

When the field of application is determined on the basis of, for example, the type of scope, the scope-type identification device 22 of the processor 2 of FIG. 1 obtains, from the memory 12 of the endoscope 1, identification information, such as a scope ID, that identifies a scope. The processor 2 associates the identification information such as a scope ID (not illustrated) with the type of the endoscope 1 and the field of application (internal medicine/surgery), and holds them in a memory (not illustrated in FIG. 1), the type of the endoscope 1 and the field of application being identified by the identification information such as the scope ID. The scope-type identification device 22 refers to information held in the memory (not illustrated), determines whether the endoscope 1 connected to the processor 2 is going to be used in internal medicine or in surgery, and reports a determination result to the application-sensitivity determination device 24. Alternatively, the scope-type identification device 22 may determine that the field of application is "surgery", by confirming that a camera head is connected to the endoscope 1.

When the field of application is determined on the basis of the content of an examination order, the examination-order identification device 23 of the processor 2 obtains necessary information from an examination order storage 41 that is included in the server 4 connected to the processor 2 through a network, the examination order storage 41 storing an examination order. The examination-order identification device 23 determines the field of application on the basis of the obtained information. The field of application may be included in the information obtained from the server 4, or the field of application may be associated with the obtained information (such as a type of examination and an examination region) and held in, for example, a memory, so as to be determined by the processor 2 by referring to the memory. The examination-order identification device 23 reports the determined field of application to the application-sensitivity determination device 24.

The operation of the application-sensitivity determination device 24 after the field of application of the endoscope 1 is reported from the scope-type identification device 22 or from the examination-order identification device 23 is similar to the operation described above. Such a configuration also permits a user to simply set an appropriate detection sensitivity of the touch panel 85 according to, for example, the usage environment of the endoscopic system 100, as in the case above.

### (Modification 2)

In the processor 2 having a configuration of FIG. 1, user-setting information is recorded in the user-setting-information recorder 30 in the processor 2 in advance. Then, the user-setting information associated with a user is read from the user-setting-information recorder 30 and reported to the application-sensitivity determination device 24. On the other hand, the present modification is different in that user-setting information recorded in an external device is read to be used.

In the present modification, the processor 2 includes a user-setting-information receiver instead of the user-setting-information recorder 30. The user-setting-information receiver receives user-setting information from a user-setting-information recorder in a portable recording medium such as a USB (universal serial bus) memory or in an external device such as a server. The user-setting-information receiver reports, to the application-sensitivity determination device 24, the user-setting information received from an external device such as an external memory or the server 4. The subsequent operation of each component is similar to that in the embodiment described above.

Such a configuration also provides an advantage similar to that provided by the above-described configuration in which the user-setting-information recorder 30 is included in the processor 2.

### <Second Embodiment>

In the embodiment described above, the user-setting information includes information that indicates whether the field of application is internal medicine or surgery, and the detection sensitivity of the touch panel 85 is set on the basis of the information on the field of application. On the other hand, in the present embodiment, a value to be set as the detection sensitivity of the touch panel 85 is recorded in the user-setting information. Using this information, the detection sensitivity of the touch panel 85 is set appropriately for each user.

The processor according to the present embodiment is described below, focusing on the difference from the embodiment described above.

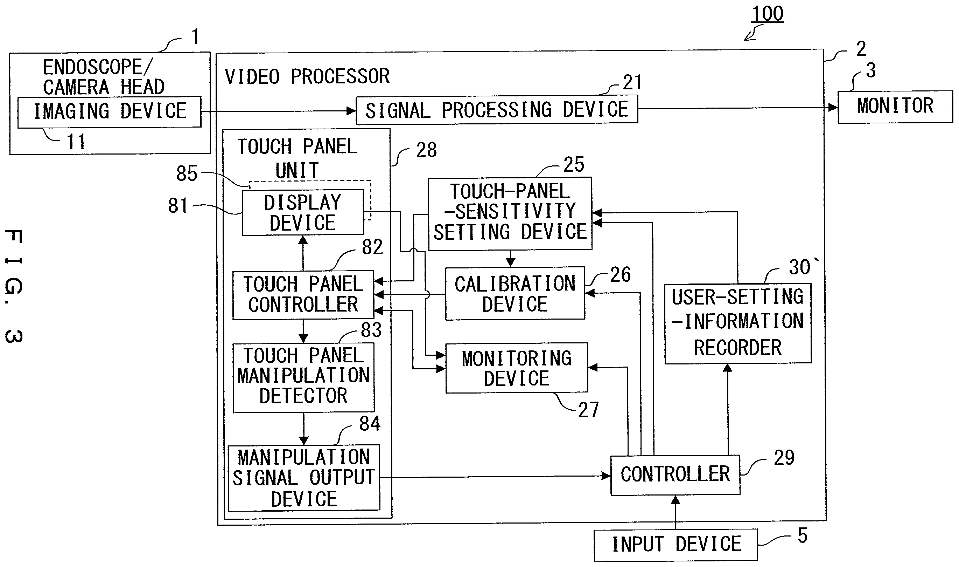

FIG. 3 illustrates a configuration of the endoscopic system that includes the processor according to the present embodiment. It is different from that of the first embodiment illustrated in FIG. 1 in that it does not include the application-sensitivity determination device 24 and in that the user-setting information recorded in the user-setting-information recorder 30 is input to the touch-panel-sensitivity setting device 25. Further, it is also different in that a value that is to be set as the detection sensitivity of the touch panel 85 is associated with a user and recorded in a user-setting-information recorder 30' as user-setting information.

In the present embodiment, at a timing such as at startup or upon starting an examination, the controller 29 reads user-setting information associated with a user from the user-setting-information recorder 30' and reports the read user-setting information to the touch-panel-sensitivity setting device 25. The touch-panel-sensitivity setting device 25 sets the detection sensitivity of the touch panel 85 on the basis of the value input from the user-setting-information recorder 30'.

In the present embodiment, the touch-panel-sensitivity setting device 25 sets the detection sensitivity of the touch panel 85 on the basis of the value that is set to be user-setting information associated with a user, the value being included in the user-setting-information recorder 30' . This makes it possible to set an appropriate value according to the individual differences in the touch panel sensitivity of a user.

For example, the user-setting information may be configured to store a value indicating that the detection sensitivity of the touch panel 85 is "high", "medium", or "low". Alternatively, it may be configured to store a sensitivity value itself.

Further, also in the present embodiment, it is possible to provide a configuration in which the detection sensitivity of the touch panel 85 can be changed by a user operating, for example, the input device 5, as in the first embodiment described above. Such a configuration also makes it possible to set the detection sensitivity of the touch panel 85 more appropriately for each user, as in the case of the first embodiment described above.

The processor 2 according to the present embodiment may also be configured to include the scope-type identification device 22 and the examination-order identification device 23, as in modification 1 of the first embodiment described above, although such a description has been omitted in FIG. 3. The field of application of the endoscope 1 is determined according to the type of scope of the endoscope 1 or the content of an examination order using the scope-type identification device 22 or the examination-order identification device 23, respectively, which also provides an advantage similar to modification 1 of the first embodiment described above.

Further, also in the present embodiment, it is possible to provide a configuration in which user-setting information is read from a USB memory or an external device such as the server 4 to be used, as in modification 2 of the first embodiment described above.

### (Function to Determine False Detection)

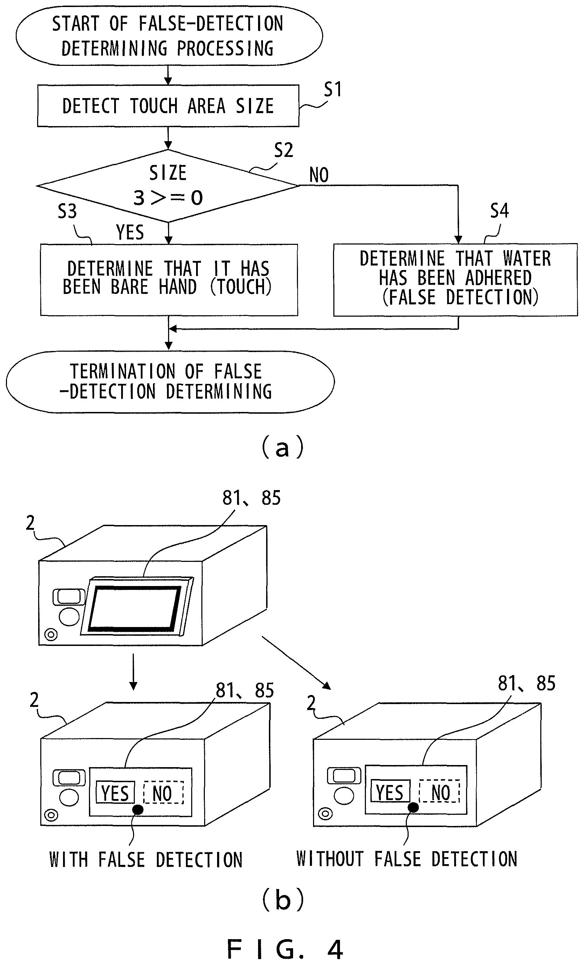

In particular, when the field of application is internal medicine, processing of determining a false detection may further be performed on the basis of a touch area size, in order to prevent the processor 2 from malfunctioning due to an adhered medicine or the like being falsely detected as a touch manipulation. This is described with reference to FIG. 4.

FIG. 4 is a diagram that explains a method for determining a false detection that is executed by the processor 2. FIG. 4(a) is a flowchart that illustrates false-detection determining processing, and FIG. 4(b) is a diagram that explains how to use a result of the false-detection determining processing of FIG. 4(a). The method for determining a false detection illustrated in FIG. 4 is applicable to both of the processors 2 according to the first and second embodiments.

When a touch position is detected in the touch panel controller 82 due to a change in capacitance of the touch panel 85, the controller 29 performs a series of false-detection determining processing illustrated in FIG. 4 (a) . First, in Step S1, the controller 29 detects a touch area size. The touch area size is the area of a portion in which a touch has been detected on the touch panel 85. The controller 29 detects the touch area size on the basis of information reported by the touch panel controller 82 of the touch panel unit 28.

In Step S2, the controller 29 determines whether the detected touch area size is greater than or equal to a predetermined threshold. The threshold is set on the basis of, for example, an average contact area of a finger when the touch panel 85 is touched, which is "3" in the example.

When the touch area size is greater than or equal to the predetermined threshold, the process moves on to Step S3 from Step S2, and the controller 29 determines that the user has pressed the touch panel 85 with the finger. On the other hand, when the touch area size is less than the predetermined threshold, the process moves on to Step S4 from Step S2, and the controller 29 determines that, for example, water or a medicine has been adhered, that is, the controller 29 determines that a touch has been falsely detected. Accordingly, the false-detection determining processing is terminated.

As illustrated in FIG. 4(b), when the touch area size is not less than the predetermined threshold, a touch detected in the touch panel unit 28 has been detected falsely, and the controller 29 of the processors 2 performs control such that particular processing is not performed. When the touch area size is greater than or equal to the predetermined threshold, the controller 29 controls each component such that processing corresponding to the detected position is performed.

As described above, filtering may be performed on the basis of a touch area size to discriminate a manipulation of a user from an attachment of a foreign substance such as water or a medicine, so as to determine a false detection. This makes it possible to effectively prevent the processor 2 from performing an operation that is not expected by the user due to, for example, a medicine or water adhered to the touch panel 85.

### (Calibration Function)

In an endoscopic examination or an endoscopic surgery, a foreign substance such as water, a medicine, or blood may be adhered to (the display part 81 of) the processor 2. The processor 2 according to the first or second embodiment described above makes it possible to deal with, for example, a problem such as is likely to occur in an examination or a surgery, so a configuration in which calibration is executed when the finger of a user leaves the touch panel 85 may be provided.

Specifically, in the processor 2 in FIG. 1 or 3, when the touch panel controller 82 of the touch panel unit 28 detects that a user has pressed on the touch panel 85, the manipulation signal output device 84 receives a report through the touch panel manipulation detector 83. The manipulation signal output device 84 of the touch panel unit 28 outputs, to the controller 29, a manipulation signal that corresponds to the received report. On the basis of the manipulation signal input from the manipulation signal output device 84 of the touch panel unit 28, the controller 29 detects that the finger of the user has left the touch panel 85. Accordingly, the controller 29 instructs the calibration device 26 to execute calibration when the finger has left the touch panel 85. The calibration device 26 performs scanning with respect to the touch panel controller 82 to obtain a capacitance (of each coordinate) of the entirety of the touch panel 85, so as to update the threshold.

In general, in the processor 2, scanning is performed and the threshold is also set at startup. However, for example, if a certain portion of the touch panel 85 has already been touched when the processor 2 is started, a value that is different from a value of an untouched other portion will be set to be a threshold of the certain portion. This makes it impossible to detect a touch on a portion for which a different threshold has been set.

In an endoscopic examination or an endoscopic surgery, a user often moves or works around the processor 2, so the problem described above is likely to occur. However, if calibration is executed when the finger of the user leaves the touch panel 85 while using the processor 2, it is possible to reset the threshold even while using the processor 2 during an examination or a surgery. Accordingly, even if a user or an object such as equipment has been in contact with the touch panel 85 when scanning is performed at startup or at the other timing, it is possible to overcome a problem in which it is not possible to detect a touch on the contacted portion throughout an examination or a surgery. A medical device such as the processor 2 is used in an examination or a surgery, so when it is used, it is generally set to be powered off after several hours have elapsed since it was powered on at startup. Thus, an execution of calibration at the timing described above is particularly effective.

Further, as a method for receiving various manipulations of a user, there is a method for assigning a predetermined function to a long-press action of the touch panel 85. For example, if a configuration in which a long-press action is detected when the touch panel 85 is touched two seconds or more is provided, it is not possible to use a method for performing scanning for every predetermined time period shorter than that, such as for each second. The reason is that it is not possible to detect a manipulation of a long-press action if scanning is performed while a user is taking a long-press action. However, if the processor 2 is configured to perform scanning when the finger of the user leaves the touch panel 85, it also becomes possible to detect a long-press action.

Furthermore, if a foreign substance such as water, medicine, or blood is adhered to the display part 81 of the touch panel unit 28, the processor 2 may detect it falsely and malfunction, but scanning at the timing described above makes it possible to avoid such a malfunction effectively. In other words, when a foreign substance has been adhered, a user touches any portion other than a portion to which the foreign substance has been adhered and leaves his/her finger, and scanning is then performed, so as to update the thresholds of the entirety of the touch panel 85. A corresponding threshold is also set for the portion to which the foreign substance has been adhered, so it is possible to avoid false detection due to the foreign substance.

### (Monitoring Function)

Further, the processors 2 according to the first and second embodiments described above may be configured to monitor the touch panel unit 28, so as to reset the touch panel unit 28 or to switch the power between off and on as appropriate when an input of a manipulation performed by a user is not allowed to be received.

Specifically, the monitoring device 27 determines, on the basis of the report from the controller 29, whether a manipulation signal from the manipulation signal output device 84 of the touch panel unit 28 has been detected in the controller 29. In FIG. 1, this determination is performed using a timer (not illustrated in FIG. 1) periodically. When it determines that a predetermined manipulation signal has not been detected from (the manipulation signal output device 84 of) the touch panel unit 28 within a predetermined time period during which the timer performs measurement, the monitoring device 27 transmits a reset signal to the touch panel unit 28 first. Then, the monitoring device 27 causes the touch panel unit 28 to perform reset processing. After that, the monitoring device 27 further performs monitoring, and when a manipulation signal is still not input to the controller 29, the monitoring device 27 transmits an instruction signal to the touch panel unit 28 so as to switch the power between off and on. When the touch panel controller 82 has been freezing due to, for example, software provided in the touch panel unit 28 going out of control, it is possible to return the touch panel unit 28 to a normal state by performing reset processing or by switching the power between off and on.

Further, when there exists an anomaly in a video displayed on the display part 81 of the touch panel unit 28, a false instruction is output to, for example, the endoscope 1, and an unexpected operation may be performed. Thus, the monitoring device 27 may be configured to also monitor the state of the display part 81, that is, a video signal input to the display part 81, and to not receive a manipulation performed on the touch panel 85 when an anomaly in a video signal has been detected. This results in effectively preventing a user from, for example, erroneously pressing a button that operates an electrocautery scalpel because a proper screen has not been displayed due to an anomaly in a video.

The present invention is not limited to the above-described embodiments as they are, but may be embodied by deforming constituents within a scope not deviating from the gist of the invention at an execution step. In addition, various inventions can be made by appropriately combining a plurality of constituents that have been disclosed in the above embodiments. For example, all the constituents that have been disclosed in the embodiments may be appropriately combined. Further, constituents in different embodiments may be appropriately combined. It should be understood that various modifications and applications can be made without departing from the scope and the spirit of the invention.

## Claims

1. An endoscopic device comprising:
an endoscopic video processor;
a touch panel provided in the endoscopic video processor; and
a touch-panel-sensitivity setting device that sets, on the basis of input identification information, a detection sensitivity of a touch manipulation performed on the touch panel.

2. The endoscopic device according to claim 1, wherein
the touch-panel-sensitivity setting device determines, on the basis of the identification information, whether the field of application in which the endoscopic video processor is used is internal medicine or surgery, and when a determination result is surgery, the touch-panel-sensitivity setting device sets the detection sensitivity to a sensitivity relatively higher than a sensitivity that is set when the determination result is internal medicine.

3. The endoscopic device according to claim 2, further comprising a scope identification device that determines a type of a scope connected to the endoscopic video processor on the basis of information input from the scope, wherein
the touch-panel-sensitivity setting device sets the detection sensitivity on the basis of the identification information generated on the basis of a result of the determination performed by the scope identification device.

4. The endoscopic device according to claim 3, wherein
the scope identification device determines the type of the scope on the basis of data output from a memory provided in the scope.

5. The endoscopic device according to claim 1, further comprising a user-setting-information recorder that records user-setting information associated with a user who uses the endoscopic video processor, wherein
the touch-panel-sensitivity setting device sets the detection sensitivity on the basis of a field of the user that is included in the user-setting information that is the identification information.

6. The endoscopic device according to claim 1, further comprising a user-setting-information recorder that records user-setting information associated with a user who uses the endoscopic video processor, wherein
the touch-panel-sensitivity setting device sets the detection sensitivity on the basis of a set value of the detection sensitivity that is included in the user-setting information that is the identification information.

7. The endoscopic device according to claim 1, further comprising an examination identification device that obtains the identification information from information input from a server in which information on an endoscopic examination has been recorded.

8. The endoscopic device according to claim 1, wherein
the touch-panel-sensitivity setting device sets the detection sensitivity to a maximum value that is allowed to be set when surgery is set to be a field of application by the setting device.

9. The endoscopic device according to claim 1, further comprising a controller, wherein when internal medicine is set to be a field of application by the setting device, the controller detects a touch area size of the touch panel, determines whether the detected touch area size is greater than or equal to a predetermined threshold, and determines that a touch on the touch panel has been falsely detected when the touch area size is less than the predetermined threshold.
